# EUROPEAN PATENT SPECIFICATION

(11) **EP 4 448 050 B1**
(45) Date of publication and mention of the grant of the patent: **19.08.2026**
(21) Application number: 22908311.8
(22) Date of filing: 13.12.2022
(51) Int. Cl.: A61M 5/142, A61M 5/14, A61M 5/00, A61M 5/168, A61M 5/172, A61M 5/175

(54) **INTERLOCK FOR MEDICAL INJECTOR METERING PUMP**
VERRIEGELUNG FÜR MESSPUMPE EINES MEDIZINISCHEN INJEKTORS
VERROUILLAGE POUR POMPE DE DOSAGE D'INJECTEUR MÉDICAL

(30) Priority: 13.12.2021 US 202163288973 P
(43) Date of publication of application: 23.10.2024
(73) Proprietor: Becton, Dickinson and Company, Franklin Lakes, NJ 07417 (US)
(72) Inventor: COLEMAN, David, James, Shankill, County Dublin D18X9Y0 (IE)
(74) Representative: Germain Maureau
(86) International application number: PCT/US2022/052669
(87) International publication number: WO 2023/114189

(56) References cited:
- WO-A1-2021/202737
- US-A- 5 494 420
- US-A- 5 741 126
- US-A- 5 832 959
- US-A1- 2010 303 655
- US-A1- 2011 245 781
- US-A1- 2013 261 599
- US-A1- 2017 067 568
- US-A1- 2017 184 091
- US-A1- 2017 184 091

## Description

### CROSS-REFERENCE TO RELATED APPLICATION

The present application claims priority to United States Provisional Application Serial No. 63/288,973, entitled "Interlock for Medical Injector Metering Pump" filed December 13, 2021.

### BACKGROUND OF THE INVENTION

### Field of the Invention

The present disclosure relates to an interlock for a medical injector metering pump.

### Description of Related Art

Wearable medical devices, such as automatic injectors, have the benefit of providing therapy to the patient at a location remote from a clinical facility and/or while being worn discretely under the patient's clothing. The wearable medical device can be applied to the patient's skin and configured to automatically deliver a dose of a pharmaceutical composition within a predetermined time period after applying the wearable medical device to the patient's skin, such as after a 27 hour delay. After the device delivers the pharmaceutical composition to the patient, the patient may subsequently remove and dispose of the device. It is known from the document US2017184091 a rotational metering pump for insulin patch. The document US2011245781 discloses a dosing unit for an ambulatory infusion device. The document US5832959 discloses a stopcock. It is further known from the document US2017067568 a stopcock with detents.

### SUMMARY OF THE INVENTION

In one aspect or embodiment according to the invention, a metering pump for a medical injector including a reservoir and a cannula, includes a housing, a sleeve at least partially received within the housing, and a piston at least partially received within the sleeve, with the piston and the sleeve defining a chamber, the piston having a first position where the chamber has a first volume and a second position where the chamber has a second volume. The first volume is larger than the second volume. The sleeve has a first rotational position where an inlet is in fluid communication with the chamber, a second rotational position where an outlet is in fluid communication with the chamber, and a third rotational position where the inlet and outlet are isolated from the chamber. The metering pump further includes an interlock member configured to restrict movement of the sleeve until the sleeve overcomes a predetermined torque value, where the interlock member is formed integrally with a portion of the housing.

The piston is configured to rotate and axially move relative to the housing and the sleeve, where the piston is configured to rotate together with the sleeve relative to the housing. The piston may be connected to the sleeve via a pin received within a helical groove defined by the sleeve. The inlet may be configured to be in fluid communication with the reservoir of the medical injector, where the outlet is configured to be in fluid communication with the cannula of the medical injector. Rotation of the piston in a first rotational direction may be configured to aspirate a fluid within the chamber and move the sleeve from the first rotational position to the second rotational position, and rotation of the piston in a second rotational direction may be configured to pump a fluid within the chamber and move the sleeve from the second rotational position to the first rotational position, with the second rotational direction opposite from the first rotational direction.

The sleeve includes a detent and the interlock member includes a protrusion, with the detent configured to deflect the protrusion and rotate past the protrusion when the sleeve overcomes the predetermined torque value. The interlock member includes a first arm extending from the housing and a second arm extending from the housing, with the protrusion positioned between the first and second arms. The first and second arms may be configured to allow movement of the protrusion between a deflected position and an undeflected position, where the first and second arms bias the protrusion toward to the undeflected position. The first and second arms may each include a recessed portion where the first and second arms extend from the housing. The first and second arms may each include a curved portion where the first and second arms extend from the protrusion. The first and second arms may each be L-shaped. The interlock member may include a living hinge.

The predetermined torque value may be 5-15 millinewton meters. The predetermined torque value may be 5-9 millinewton meters.

### BRIEF DESCRIPTION OF THE DRAWINGS

The above-mentioned and other features and advantages of this disclosure, and the manner of attaining them, will become more apparent and the disclosure itself will be better understood by reference to the following descriptions of embodiments of the disclosure taken in conjunction with the accompanying drawings, wherein:
FIG. 1 is a perspective view of a conventional interlock and metering pump assembly;
FIG. 2 is a perspective view of the interlock and metering pump assembly of FIG. 1, showing a ready to dispense stage of operation;
FIG. 3 is a perspective view of the interlock and metering pump assembly of FIG. 1, showing a ready to aspirate stage of operation;
FIG. 4 is a schematic view of a conventional medical injector;
FIG. 5 is a perspective view of an interlock and metering pump assembly according to one aspect or embodiment of the present application, showing an undeflected position of the interlock; and
FIG. 6 is a perspective view of an interlock and metering pump assembly according to one aspect or embodiment of the present application, showing a deflected position of the interlock.

Corresponding reference characters indicate corresponding parts throughout the several views. The exemplifications set out herein illustrate exemplary embodiments of the disclosure, and such exemplifications are not to be construed as limiting the scope of the disclosure in any manner.

### DETAILED DESCRIPTION OF THE INVENTION

Spatial or directional terms, such as "left", "right", "inner", "outer", "above", "below", and the like, are not to be considered as limiting as the invention can assume various alternative orientations.

All numbers used in the specification and claims are to be understood as being modified in all instances by the term "about". By "about" is meant a range of plus or minus ten percent of the stated value. As used in the specification and the claims, the singular form of "a", "an", and "the" include plural referents unless the context clearly dictates otherwise. The terms "first", "second", and the like are not intended to refer to any particular order or chronology, but instead refer to different conditions, properties, or elements. By "at least" is meant "greater than or equal to".

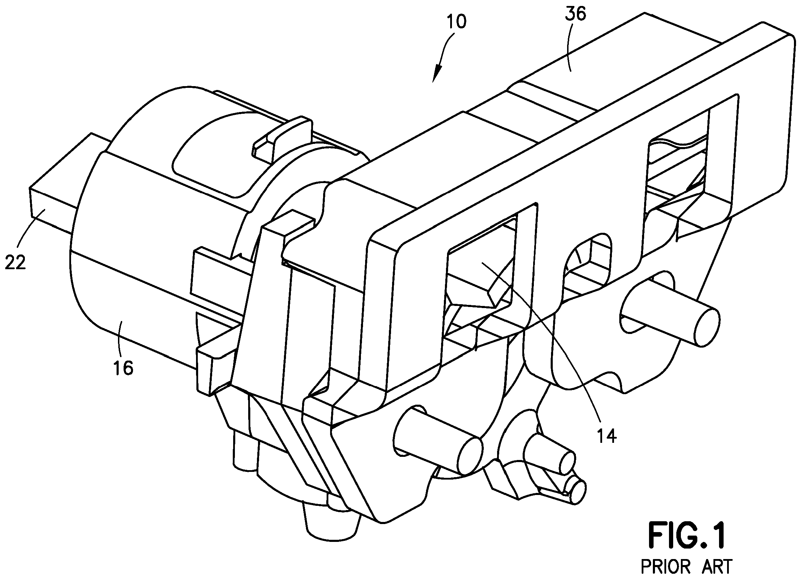

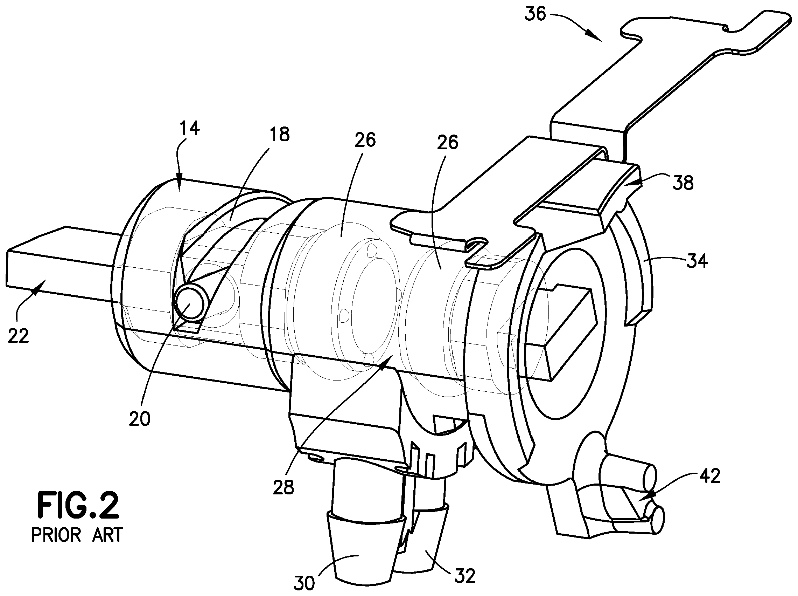

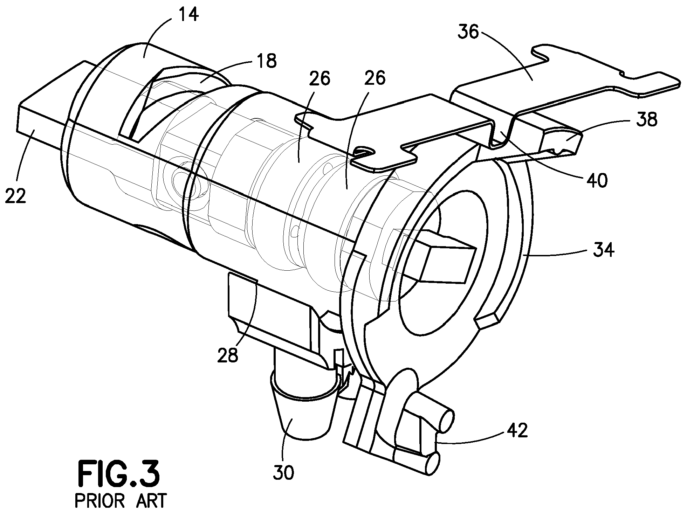

Referring to FIGS. 1-4, a conventional metering pump 10 for a medical injector or drug delivery device 12 is shown. The metering pump 10 is a rotational metering pump, which is described in International Publication No. WO 2015/157174. The metering pump 10 is configured to be connected to a DC motor and gearbox assembly (not shown) to rotate a sleeve 14 in a housing 16. A helical groove 18 is provided on the sleeve 14. A coupling pin 20 connected to a piston 22 translates along the helical groove 18 to guide the retraction and insertion of the piston 22 within the sleeve 14, respectively, as the sleeve 14 rotates in one direction and then rotates in the opposite direction. The sleeve 14 has an end plug 24. Two seals 26 on the respective ends of the piston 22 and the end plug 24 that are interior to the sleeve 14 define a cavity or chamber 28 when the piston 22 is retracted, as depicted in FIG. 2, following an aspirate stroke and therefore ready to dispense. The volume of the chamber 28 therefore changes depending on the degree of retraction of the piston 22. The volume of the chamber 28 is negligible or essentially zero when the piston 22 is fully inserted and the seals 26 are substantially in contact with each other following a dispense stroke, as depicted in FIG. 3, and therefore ready to aspirate.

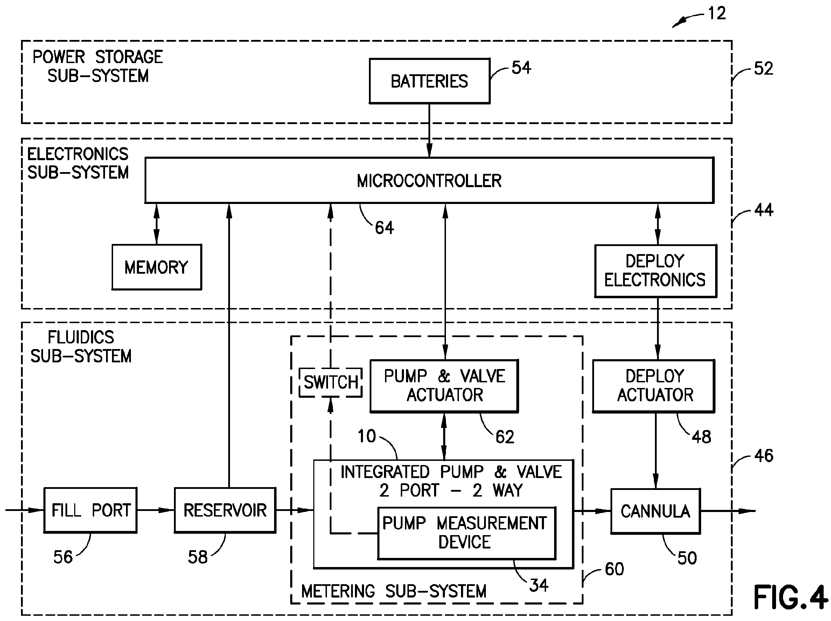

Two ports 30, 32 are provided relative to the housing 16, including an inlet port 30 through which medication can flow from a reservoir 58 (FIG. 4) for the pump 10 (FIG. 4), and an outlet port 32 through which the medication that has been drawn into the chamber 28 (*e*.*g*., by retraction of the piston 22 during an aspirate stage of operation) can be dispensed from the chamber 28 to, for example, a fluid path to a cannula 50 (FIG. 4) in the patient by re-insertion of the piston 22 into the chamber 28.

Referring to FIGS. 1-3, the sleeve 14 includes an aperture (not shown) that aligns with the outlet port 32 or the inlet port 30 (*i*.*e*., depending on the degree of rotation of the sleeve 14 and therefore the degree of translation of the piston 22 to permit the medication in the chamber 28 to flow through the corresponding one of the ports 30, 32). A pump measurement device 34 (FIG. 4) such as a sleeve rotational limit switch can be provided which has, for example, an interlock 36 and one or more detents 38 on the sleeve 14 or its end plug 24 that cooperate with the interlock 36. The interlock 36 can be mounted to the housing 16 at each end thereof. The detent 38 at the end face of sleeve 14 is adjacent to a bump 40 of the interlock 36.

Under certain conditions, such as back pressure, it is possible that friction between the piston 22 and the sleeve 14 is sufficient to cause the sleeve 14 to rotate before the piston 22 and the coupling pin 20 reach either end of the helical groove 18, which could result in an incomplete volume of liquid being pumped per stroke. In order to prevent this situation, the interlock 36 prevents the sleeve 14 from rotating until the torque passes a predetermined threshold, as shown in FIG. 2. This ensures that piston 22 fully rotates within the sleeve 14 until the coupling pin 20 reaches the end of the helical groove 18. Once the coupling pin 20 hits the end of the helical groove 18, further movement by the DC motor and gearbox assembly or other type of pump and valve actuator 62 (FIG. 4) increases torque on the sleeve 14 beyond the threshold, causing the interlock 36 to flex and permit the detent 38 to pass by the bump 40. At the completion of rotation of the sleeve 14, such that its port or aperture is oriented with the cannula or outlet port 32, the detent 38 moves past the bump 40 in the interlock 36, as shown in FIG. 3. Another sleeve feature 42 can be provided to engage an electrical switch (*e*.*g*., an end-stop switch provided on a printed circuit board and disposed relative to the sleeve 14 and/or the end plug 24 to cooperate with the pump measurement device 34 as shown in FIG. 4).

Referring to FIG. 4, the medical injector 12 may include the metering pump 10, as described above, an electronics sub-system 44 for controlling operations of components in a fluidics sub-system 46, such as the pump 10, and an insertion mechanism 48 for deploying the cannula 50 for insertion into an infusion site on a patient's skin. A power storage sub-system 52 can include batteries 54, for example, for providing power to components in the electronics and fluidics sub-systems 44, 46. The fluidics sub-system 46 can include, for example, an optional fill port 56 for filling a reservoir 58 (*e*.*g*., with medication), although the medical injector can be optionally shipped from a manufacture having its reservoir already filled. The fluidics sub-system 46 also has a metering sub-system 60 including the metering pump 10 and the pump and valve actuator 62.

As described above, the metering pump 10 can have two ports 30, 32 and related valve sub-assembly that controls when fluid enters and leaves the chamber 28 via the respective ports 30, 32. One of the ports is the inlet port 30 through which fluid such as liquid medication flows from the reservoir 58 into the metering pump 10 as the result of a pump intake or pull stroke. Fluid leaves the chamber 28 of the metering pump 10 through the outlet port 32 and flows toward the cannula 50 for administration as the result of a pump discharge or push stroke of the metering pump 10. The pump and valve actuator 62 can be a DC motor and gearbox assembly or other pump driving mechanism for controlling the plunger 22 or piston and other related pump parts, such as the sleeve 14, that may rotate relative to the translational movement of the piston 22. A microcontroller 64 can be provided with an integrated or separate memory device having computer software instructions to actuate, for example, rotation of the sleeve 14 in a selected direction, translational or axial movement of the piston 22 in the sleeve 14 for an aspirate or dispense stroke, and optionally the rotation of the sleeve 14 and piston 22 together during a valve state change as described in the above-referenced WO 2015/157174. The metering pump 10 and the interlock 36 may be the same as the metering pump and interlock shown and described in International Publication No. WO 2019/156848.

The interlock 36 of the metering pump 10 shown in FIGS. 1-3 is formed separately from the housing 16. In particular, the interlock 36 is formed from sheet metal and attached to the housing 16. Due to manufacturing tolerances and other variability, the performance of the interlock 36 can be inconsistent between the aspirate and dispense movements and can also be inconsistent from device to device, which can result in different force profiles or patterns between devices that makes detecting occlusions more difficult. Forming the interlock 36 separately also requires the manufacture of a separate part and a subsequent assembly step.

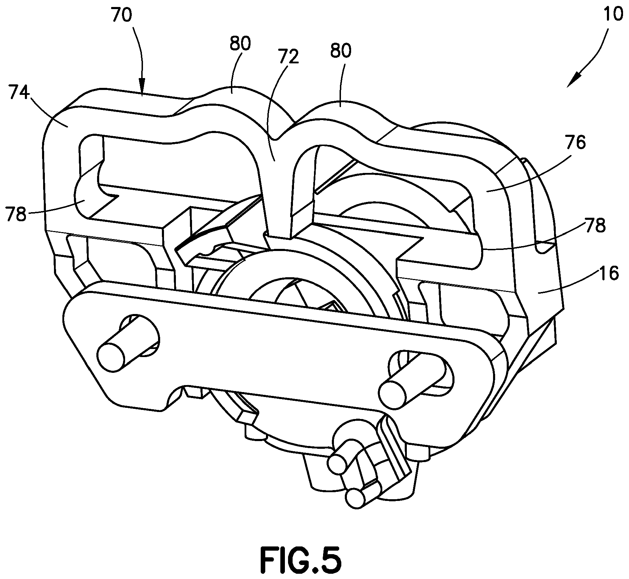

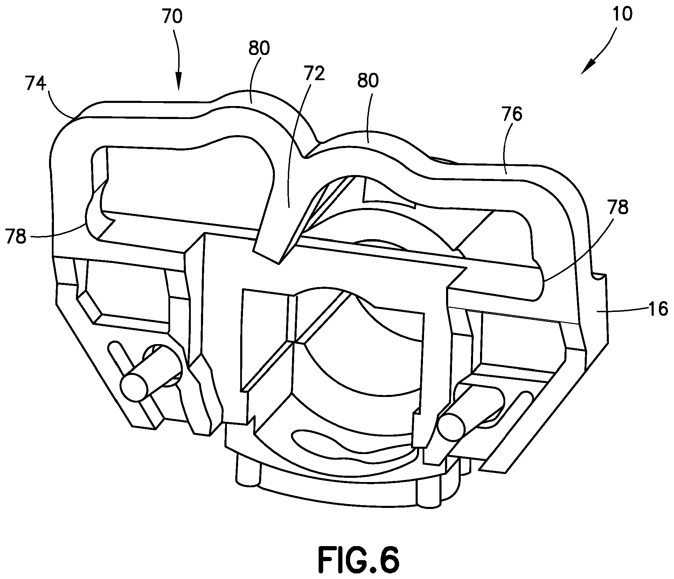

Referring to FIGS. 5 and 6, an interlock member 70 for the metering pump 10 according to one aspect or embodiment of the present application is shown. In one aspect or embodiment, the interlock member 70 replaces the interlock 36 of the metering pump 10 of FIGS. 1-4, with the metering pump 10 otherwise unchanged and performing as described above.

In one aspect or embodiment, the metering pump 10 includes the housing 16, the sleeve 14 at least partially received within the housing 16, and the piston 22 at least partially received within the sleeve 14, with the piston 22 and the sleeve 14 defining the chamber 28. As described above, the piston 22 has first position where the chamber 28 has a first volume and a second position where the chamber 28 has a second volume, where the first volume is larger than the second volume. The sleeve 14 has a first rotational position where the inlet port 30 is in fluid communication with the chamber 28, a second rotational position where the outlet port 32 is in fluid communication with the chamber 28, and a third rotational position where the inlet port 30 and outlet port 32 are isolated from the chamber 28. The metering pump 10 also includes the interlock member 70 configured to restrict movement of the sleeve 14 until the sleeve 14 overcomes a predetermined torque value, with the interlock member 70 formed integrally with a portion of the housing 16. In one aspect or embodiment, the interlock member 70 is molded together and from the same material as the housing 16. In one aspect or embodiment, the housing 16 and the interlock member 70 are formed from plastic.

Rotation of the piston 22 in a first rotational direction is configured to aspirate a fluid within the chamber 28 and move the sleeve 14 from the first rotational position to the second rotational position. Rotation of the piston 22 in a second rotational direction is configured to pump a fluid within the chamber 28 and move the sleeve 14 from the second rotational position to the first rotational position. The second rotational direction is opposite from the first rotational direction.

Referring again to FIGS. 5 and 6, the sleeve 14 includes the detent 38 and the interlock member 70 includes a protrusion 72, with the detent 38 configured to deflect the protrusion 72 and rotate past the protrusion 72 when the sleeve 14 overcomes the predetermined torque value. More specifically, the piston 22 rotates and axially moves within the sleeve 14 to aspirate or pump fluid from the chamber 28 while the sleeve 14 rotates to control when the chamber 28 is in fluid communication with the inlet port 30 and outlet port 32. The interlock member 70 prevents the sleeve 14 from rotating to change the valve state of the pump 10 until the coupling pin 20 reaches the end of the helical groove 18 and the pump and valve actuator 62 can apply a torque to the sleeve 14 via the pin 20 that is higher than the predetermined torque value. Once the torque exceeds the predetermined torque value, the detent deflects and pushes past the protrusion 72 of the interlock member 70 thereby allowing the sleeve 14 to rotate and change the valve state of the metering pump 10.

The interlock member 70 includes a first arm 74 extending from the housing 16 and a second arm 76 extending from the housing 16, with the protrusion 72 positioned between the first and second arms 74, 76. The first and second arms 74, 76 are configured to allow movement of the protrusion 72 between a deflected position (FIG. 6) and an undeflected position (FIG. 5). The first and second arms 74, 76 bias the protrusion 72 toward to the undeflected position. The first and second arms 74, 76 each include a recessed portion 78 where the first and second arms 74, 76 extend from the housing 16 and a curved portion 80 where the first and second arms 74, 76 extend from the protrusion 72. The recessed portions 78 and the curved portions 80 are configured to allow the interlock member 70 to flex when the protrusion 72 engages the detent 38. The first and second arms 74, 76 may each be L-shaped, although other suitable shapes and arrangements may be utilized. In one aspect or embodiment, the first interlock member 70 includes a living hinge or living spring.

In one aspect or embodiment, the predetermined torque value for overcoming the interlock member 70 is 5-15 millinewton meters. In a further aspect or embodiment, the predetermined torque value for overcoming the interlock member 70 is 5-9 millinewton meters.

The interlock member 70 of FIGS. 5 and 6 is configured to reduce performance variation of the interlock member 70 between devices and between aspiration and dispensing strokes thereby improving occlusion detection reliability. The interlock member 70 of FIGS. 5 and 6 reduces the part count of the metering pump 10 and assembly complexity. Further, the interlock member 70 is configured to reduce the operating noise of the interlock member 70 compared to the interlock 36 of FIGS. 1-3.

Although the invention has been described in detail for the purpose of illustration based on what is currently considered to be the most practical and preferred embodiments, it is to be understood that such detail is solely for that purpose and that the invention is not limited to the disclosed embodiments, but, on the contrary, is intended to cover modifications that are within the scope of the appended claims. For example, it is to be understood that the present invention contemplates that, to the extent possible, one or more features of any embodiment can be combined with one or more features of any other embodiment.

## Claims

1. A metering pump (10) for a medical injector (12) comprising a reservoir (58) and a cannula (50), the metering pump (10) comprising:
a housing (16);
a sleeve (14) at least partially received within the housing (16), the sleeve (14) comprising a detent (38);
a piston (22) at least partially received within the sleeve (14), the piston (22) and the sleeve (14) defining a chamber (28), the piston (22) having a first position where the chamber (28) has a first volume and a second position where the chamber (28) has a second volume, the first volume larger than the second volume, the sleeve (14) having a first rotational position where an inlet (30) is in fluid communication with the chamber (28), a second rotational position where an outlet (32) is in fluid communication with the chamber (28), and a third rotational position where the inlet (30) and outlet (32) are isolated from the chamber (28), the piston (22) is configured to rotate and axially move relative to the housing (16) and the sleeve (14) and is also configured to rotate together with the sleeve (14) relative to the housing (16); and
an interlock member (70) configured to restrict movement of the sleeve (14) until the sleeve (14) overcomes a predetermined torque value, the interlock member (70) comprising a protrusion (72), the detent (38) of the sleeve (14) configured to deflect the protrusion (72) and rotate past the protrusion (72) when the sleeve (14) overcomes the predetermined torque value,
**characterized in that** the interlock member (70) is formed integrally with a portion of the housing (16), and **in that** interlock member (70) comprises a first arm (74) extending from the housing (16) and a second arm (76) extending from the housing (16), with the protrusion (72) positioned between the first and second arms (74,76).

2. The pump of claim 1, wherein the piston (22) is connected to the sleeve (14) via a pin (20) received within a helical groove (18) defined by the sleeve (14).

3. The pump of claim 2, wherein the inlet (30) is configured to be in fluid communication with the reservoir (58) of the medical injector (12), and wherein the outlet (32) is configured to be in fluid communication with the cannula (50) of the medical injector (12).

4. The pump of claim 3, wherein rotation of the piston (22) in a first rotational direction is configured to aspirate a fluid within the chamber (28) and move the sleeve (14) from the first rotational position to the second rotational position, and wherein rotation of the piston (22) in a second rotational direction is configured to pump a fluid within the chamber (28) and move the sleeve (14) from the second rotational position to the first rotational position, the second rotational direction being opposite from the first rotational direction.

5. The pump of claim 1, wherein the first and second arms (74,76) are configured to allow movement of the protrusion (72) between a deflected position and an undeflected position, and wherein the first and second arms (74,76) bias the protrusion (72) toward the undeflected position.

6. The pump of claim 5, wherein the first and second arms (74,76) each comprise a recessed portion (78) where the first and second arms (74,76) extend from the housing (16).

7. The pump of claim 5 or claim 6, wherein the first and second arms (74,76) each comprise a curved portion (80) where the first and second arms (74,76) extend from the protrusion (72).

8. The pump of any of claims 1-7, wherein the first and second arms (74,76) are each L-shaped.

9. The pump of any of claims 1-8, wherein the interlock member (70) comprises a living hinge.

10. The pump of any of claims 1-9, wherein the predetermined torque value is 5-15 millinewton meters.

11. The pump of any of claims 1-9, wherein the predetermined torque value is 5-9 millinewton meters.

## Patentansprüche

1. Messpumpe (10) für eine medizinische Injektionsvorrichtung (12), die ein Reservoir (58) und eine Kanüle (50) umfasst, wobei die Messpumpe (10) umfasst:
ein Gehäuse (16);
eine Hülse (14), die mindestens teilweise innerhalb des Gehäuses (16) aufgenommen ist, wobei die Hülse (14) eine Raste (38) umfasst;
einen Kolben (22), der mindestens teilweise innerhalb der Hülse (14) aufgenommen ist, wobei der Kolben (22) und die Hülse (14) eine Kammer (28) definieren, wobei der Kolben (22) eine erste Position aufweist, in der die Kammer (28) ein erstes Volumen aufweist, und eine zweite Position, in der die Kammer (28) ein zweites Volumen aufweist, wobei das erste Volumen größer ist als das zweite Volumen, wobei die Hülse (14) eine erste Drehposition aufweist, in der ein Einlass (30) mit der Kammer (28) in strömungstechnischer Kommunikation steht, eine zweite Drehposition, in der ein Auslass (32) mit der Kammer (28) in strömungstechnischer Kommunikation steht, und eine dritte Drehposition, in der der Einlass (30) und der Auslass (32) von der Kammer (28) isoliert sind, wobei der Kolben (22) so ausgestaltet ist, dass er sich relativ zu dem Gehäuse (16) und der Hülse (14) dreht und axial bewegt, und auch so ausgestaltet ist, dass er sich zusammen mit der Hülse (14) relativ zu dem Gehäuse (16) dreht; und
ein Verriegelungselement (70), das so ausgestaltet ist, dass es Bewegung der Hülse (14) einschränkt, bis die Hülse (14) einen vorbestimmten Drehmomentwert überwindet, wobei das Verriegelungselement (70) einen Vorsprung (72) umfasst, wobei die Raste (38) der Hülse (14) so ausgestaltet ist, dass sie den Vorsprung (72) auslenkt und an dem Vorsprung (72) vorbeidreht, wenn die Hülse (14) den vorbestimmten Drehmomentwert überwindet, **dadurch gekennzeichnet, dass** das Verriegelungselement (70) einstückig mit einem Abschnitt des Gehäuses (16) gebildet ist, und dass das Verriegelungselement (70) einen sich aus dem Gehäuse (16) erstreckenden ersten Arm (74) und einen sich aus dem Gehäuse (16) erstreckenden zweiten Arm (76) umfasst, wobei der Vorsprung (72) zwischen dem ersten und dem zweiten Arm (74, 76) positioniert ist.

2. Pumpe nach Anspruch 1, wobei der Kolben (22) über einen Stift (20), der in einer durch die Hülse (14) definierten spiralförmigen Nut (18) aufgenommen ist, mit der Hülse (14) verbunden ist.

3. Pumpe nach Anspruch 2, wobei der Einlass (30) so ausgestaltet ist, dass er mit dem Reservoir (58) der medizinischen Injektionsvorrichtung (12) in strömungstechnischer Kommunikation steht, und wobei der Auslass (32) so ausgestaltet ist, dass er mit der Kanüle (50) der medizinischen Injektionsvorrichtung (12) in strömungstechnischer Kommunikation steht.

4. Pumpe nach Anspruch 3, wobei Drehung des Kolbens (22) in eine erste Drehrichtung so ausgestaltet ist, dass sie ein Fluid innerhalb der Kammer (28) ansaugt und die Hülse (14) aus der ersten Drehposition in die zweite Drehposition bewegt, und wobei Drehung des Kolbens (22) in eine zweite Drehrichtung so ausgestaltet ist, dass sie ein Fluid innerhalb der Kammer (28) pumpt und die Hülse (14) aus der zweiten Drehposition in die erste Drehposition bewegt, wobei die zweite Drehrichtung der ersten Drehrichtung entgegengesetzt ist.

5. Pumpe nach Anspruch 1, wobei der erste und der zweite Arm (74, 76) so ausgestaltet sind, dass sie Bewegung des Vorsprungs (72) zwischen einer ausgelenkten Position und einer nicht ausgelenkten Position ermöglichen, und wobei der erste und der zweite Arm (74, 76) den Vorsprung (72) in Richtung der nicht ausgelenkten Position vorspannen.

6. Pumpe nach Anspruch 5, wobei der erste und der zweite Arm (74, 76) jeweils einen vertieften Abschnitt (78) umfassen, an dem sich der erste und der zweite Arm (74, 76) aus dem Gehäuse (16) erstrecken.

7. Pumpe nach Anspruch 5 oder Anspruch 6, wobei der erste und der zweite Arm (74, 76) jeweils einen gekrümmten Abschnitt (80) umfassen, an dem sich der erste und der zweite Arm (74, 76) von dem Vorsprung (72) erstrecken.

8. Pumpe nach einem der Ansprüche 1-7, wobei der erste und der zweite Arm (74, 76) jeweils L-förmig sind.

9. Pumpe nach einem der Ansprüche 1-8, wobei das Verriegelungselement (70) ein Filmscharnier umfasst.

10. Pumpe nach einem der Ansprüche 1-9, wobei der vorbestimmte Drehmomentwert 5-15 Millinewtonmeter beträgt.

11. Pumpe nach einem der Ansprüche 1-9, wobei der vorbestimmte Drehmomentwert 5-9 Millinewtonmeter beträgt.

## Revendications

1. Pompe de dosage (10) pour un injecteur médical (12) comprenant un réservoir (58) et une canule (50), la pompe de dosage (10) comprenant :
un boîtier (16) ;
un manchon (14) reçu au moins partiellement dans le boîtier (16), le manchon (14) comprenant un ergot (38) ;
un piston (22) reçu au moins partiellement dans le manchon (14), le piston (22) et le manchon (14) définissant une chambre (28), le piston (22) ayant une première position dans laquelle la chambre (28) a un premier volume et une seconde position dans laquelle la chambre (28) a un second volume, le premier volume étant supérieur au second volume, le manchon (14) ayant une première position de rotation dans laquelle une entrée (30) est en communication fluidique avec la chambre (28), une deuxième position de rotation dans laquelle une sortie (32) est en communication fluidique avec la chambre (28), et une troisième position de rotation dans laquelle l'entrée (30) et la sortie (32) sont isolées de la chambre (28), le piston (22) est configuré pour tourner et se déplacer axialement par rapport au boîtier (16) et au manchon (14) et est également configuré pour tourner conjointement avec le manchon (14) par rapport au boîtier (16) ; et
un élément de verrouillage (70) configuré pour limiter le déplacement du manchon (14) jusqu'à ce que le manchon (14) dépasse une valeur de couple prédéterminée, l'élément de verrouillage (70) comprenant une saillie (72), l'ergot (38) du manchon (14) étant configuré pour dévier la saillie (72) et tourner au-delà de la saillie (72) lorsque le manchon (14) dépasse la valeur de couple prédéterminée,
**caractérisée en ce que** l'élément de verrouillage (70) est formé d'un seul tenant avec une partie du boîtier (16),
et **en ce que** l'élément de verrouillage (70) comprend un premier bras (74) s'étendant à partir du boîtier (16) et un second bras (76) s'étendant à partir du boîtier (16), avec la saillie (72) positionnée entre les premier et second bras (74, 76).

2. Pompe selon la revendication 1, dans laquelle le piston (22) est relié au manchon (14) par l'intermédiaire d'une goupille (20) reçue dans une rainure hélicoïdale (18) définie par le manchon (14).

3. Pompe selon la revendication 2, dans laquelle l'entrée (30) est configurée pour être en communication fluidique avec le réservoir (58) de l'injecteur médical (12), et dans laquelle la sortie (32) est configurée pour être en communication fluidique avec la canule (50) de l'injecteur médical (12).

4. Pompe selon la revendication 3, dans laquelle la rotation du piston (22) dans une première direction de rotation est configurée pour aspirer un fluide à l'intérieur de la chambre (28) et déplacer le manchon (14) de la première position de rotation à la deuxième position de rotation, et dans laquelle la rotation du piston (22) dans une seconde direction de rotation est configurée pour pomper un fluide à l'intérieur de la chambre (28) et déplacer le manchon (14) de la deuxième position de rotation à la première position de rotation, la seconde direction de rotation étant opposée à la première direction de rotation.

5. Pompe selon la revendication 1, dans laquelle les premier et second bras (74, 76) sont configurés pour permettre le déplacement de la saillie (72) entre une position déviée et une position non déviée, et dans laquelle les premier et second bras (74, 76) sollicitent la saillie (72) vers la position non déviée.

6. Pompe selon la revendication 5, dans laquelle les premier et second bras (74, 76) comprennent chacun une partie en retrait (78) dans laquelle les premier et second bras (74, 76) s'étendent à partir du boîtier (16).

7. Pompe selon la revendication 5 ou la revendication 6, dans laquelle les premier et second bras (74, 76) comprennent chacun une partie incurvée (80) dans laquelle les premier et second bras (74, 76) s'étendent à partir de la saillie (72).

8. Pompe selon l'une des revendications 1 à 7, dans laquelle les premier et second bras (74, 76) sont chacun en forme de L.

9. Pompe selon l'une des revendications 1 à 8, dans laquelle l'élément de verrouillage (70) comprend une charnière souple.

10. Pompe selon l'une des revendications 1 à 9, dans laquelle la valeur de couple prédéterminée est de 5 à 15 millinewton-mètres.

11. Pompe selon l'une des revendications 1 à 9, dans laquelle la valeur de couple prédéterminée est de 5 à 9 millinewton-mètres.
